# EUROPÄISCHE PATENTANMELDUNG

(11) **EP 3 549 554 A1**
(43) Veröffentlichungstag der Anmeldung: **09.10.2019**
(21) Anmeldenummer: 18165737.0
(22) Anmeldetag: 04.04.2018
(51) Int. Cl.: A61F 2/52

(54) **KONKAVE BRUSTPROTHESE**

(71) Anmelder: Edelweiss Basics GmbH & Co. KG, 83098 Brannenburg (DE)
(72) Erfinder: VOLK, Stephan, 83646 Bad Tölz (DE)
(74) Vertreter: Kador & Partner PartG mbB

(57) **Zusammenfassung**

Gegenstand der Erfindung ist eine symmetrische Brustprothese umfassend einen Körper, der eine die Form einer weiblichen Brust aufweisende Vorderseite und eine Rückseite aufweist, wobei die Rückseite einen linken Seitenrand, einen rechten Seitenrand, eine Breite B und eine Höhe H aufweist, wobei das Verhältnis B:H zwischen 1,05 und 1,45 liegt, und wobei der linke Seitenrand und der rechte Seitenrand jeweils einen konkaven Verlauf aufweisen und jeweils eine Konkavität s zwischen 0,5 mm und 6 mm aufweisen.

## Beschreibung

Die Erfindung betrifft eine symmetrische Brustprothese zum Tragen in der linken oder rechten Büstenschale eines Büstenhalters oder Korseletts nach einer Brustamputation bei Frauen, insbesondere eine symmetrische Brustprothese mit konkaven Seitenrändern und einem spezifischen Breite zu Höhe Verhältnis der Rückseite der Brustprothese.

Symmetrische Brustprothesen sind beispielsweise aus der DE 198 38 428 A1 bekannt. Sie hat eine symmetrische Dreiecksform und kann infolgedessen wahlweise als Ersatz für die rechte oder linke amputierte Brust getragen werden. Obwohl diese Brustprothese einen fast vollkommenen Ausgleich für das entfernte Brustgewebe bis in den Achselbereich bietet, wird sie von Prothesenträgerinnen nicht immer als optimal empfunden, weil der seitliche Ausläufer zum Brustbein zu lang sein kann und dann stört, besonders wenn die Prothese beispielsweise in einem Bügel-Büstenhalter getragen wird.

Es sind auch symmetrische Brustprothesen in sogenannter Tropfenform im Stand der Technik bekannt. Diese Prothesen zeichnen sich durch einen relativen kleinen Unterbrustradius, und damit durch eine verkleinerte Prothesenbreite in Relation zu ihrer Höhe aus. Dies ermöglicht der Trägerin, dass die tropfenförmige Brustprothese im Büstenhalter je nach Bedarf zu einem gewissen Grad gedreht werden kann. Sie hat aber den Nachteil, dass sie oberhalb der Mamma wegoperiertes Brustgewebe nicht ausgleichen und in diesem Bereich auch keinen stufenlosen Übergang zwischen der Prothesenoberfläche und dem Körper der Trägerin schaffen kann.

Die ideale Form der Brustprothese hatte bislang nur die asymmetrische Brustprothese die zwei Ausläufer aufweist, von denen der obere Ausläufer kleiner als der seitliche Ausläufer ist. Zwangsläufig sind asymmetrische Brustprothesen so ausgebildet, dass sie entweder nur auf der linken Körperseite oder nur auf der rechten Körperseite getragen werden können. Da die Formen für "linke" asymmetrische Brustprothesen verschieden von den Formen für "rechte" asymmetrische Brustprothesen sind, sind asymmetrische Brustprothesen in der Herstellung grundsätzlich teurer als symmetrische Brustprothesen.

Es ist zudem ein ständiges Bestreben im Bereich der Brustprothesen den Tragekomfort einer Brustprothese zu erhöhen und die Passform der Brustprothese zu verbessern. Der Tragekomfort erhöht sich, vor allem bei größeren Brustgrößen, beispielsweise durch ein reduziertes Gewicht der Brustprothese.

Allerdings können eine verbesserte Passform und ein reduziertes Gewicht sich diametral auf die Stabilität und das der gesunden Brust angepasste Schwingungsverhalten der Brustprothese auswirken. Üblicherweise kann eine Gewichtsreduktion der Brustprothese sich beispielsweise negativ auf das Schwingungsverhalten der Prothese auswirken, sodass bei Bewegung der Trägerin ein Unterschied im Schwingungsverhalten der gesunden Brust zur Brustprothese unerwünscht bemerkbar wird.

Auch verbesserte Passformen können sich negativ auf die strukturelle Stabilität einer Brustprothese auswirken. So kann, insbesondere beim Sport und bei Bewegung, die Brustprothese beispielsweise teilweise zusammensacken oder sich anderweitig teilweise verformen und somit eine unerwünschte, nicht der natürlichen Form der Brust entsprechende Gestalt annehmen.

Der Erfindung liegt die Aufgabe zugrunde, eine Brustprothese bereitzustellen, die die oben erwähnten Nachteile vermeidet.

Der Erfindung liegt ferner die Aufgabe zugrunde eine Brustprothese bereitzustellen, die angenehm für die Trägerin zu tragen ist, eine gute Passform aufweist und zugleich ein Schwingungsverhalten wie jenes der natürlichen gesunden Brust und die erforderliche strukturelle Stabilität aufweist.

Es ist eine weitere Aufgabe der Erfindung eine Brustprothese bereitzustellen, die im Büstenhalter sowohl links als auch rechts und in einer Vielzahl von Büstenhaltern der Trägerin getragen werden kann ohne für Dritte sichtbar zu sein.

Die Erfindung liegt die Erkenntnis zugrunde, dass alle oben erwähnten Aufgaben durch eine symmetrische Brustprothese mit konkavem Verlauf der beiden Seitenrändern sowie einem spezifischen Breite zu Höhe Verhältnis der Rückseite des Körpers der Brustprothese gelöst werden können.

Gegenstand der Erfindung ist eine symmetrische Brustprothese umfassend einen Körper, der eine die Form einer weiblichen Brust aufweisende Vorderseite und eine Rückseite aufweist, wobei die Rückseite einen linken Seitenrand, einen rechten Seitenrand, eine Breite B und eine Höhe H aufweist, wobei das Verhältnis B:H zwischen 1,05 und 1,45 liegt, und wobei der linke Seitenrand und der rechte Seitenrand jeweils einen konkaven Verlauf aufweisen und jeweils eine Konkavität s zwischen 0,5 mm und 6 mm aufweisen.

Die erfindungsgemäße Brustprothese weist zahlreiche Vorteile auf. Durch die erfindungsgemäße Konkavität der Seitenränder der Brustprothese sowie dem dadurch bedingten schlankeren oder schmäleren oberen Ausläufer wird, im Vergleich zu Stand der Technik-Prothesen mit breitem oberen Ausläufer und geraden oder gar konvexen Seitenrändern, das Volumen im oberen Drittel des Brustprothesenkörpers verringert, wodurch sich weniger Füllmasse im oberen Drittel des Körpers der Brustprothese befindet. Dadurch wird eine dem Tragekomfort dienliche Gewichtsreduktion erreicht, und gleichzeitig die erforderliche Stabilität und das Schwingungsverhalten der Prothese entsprechend einer gesunden Brust gewährleistet. Die erfindungsgemäße Brustprothese stellt überraschenderweise eine im Gewicht reduzierte Brustprothese bereit, die dennoch die erforderliche Stabilität und das natürliche Schwingungsverhalten einer gesunden Brust aufweist.

Zur gleichen Zeit weist die erfindungsgemäße Brustprothese als weiteren Vorteil aufgrund ihres speziellen Breite zu Höhe Verhältnisses und der Konkavität eine verbesserte Passform auf. Dadurch ermöglicht die erfindungsgemäße Brustprothese beispielsweise ein Tragen im Büstenhalter mit tieferem Dekolleté, ohne dass die Brustprothese von Dritten gesehen werden kann.

Es ist ein weiterer Vorteil der Erfindung, dass durch den schlankeren oder schmäleren oberen Ausläufer und der Konkavität die erfindungsgemäße Brustprothese im Büstenhalter auch zum einem gewissen Grad gedreht werden kann, beispielsweise nach außen zur Achsel hin. Damit kann ein verbesserter Versorgungsausgleich erzielt werden ohne dass die Brustprothese vom Büstenhalter nicht mehr abgedeckt wird und für Dritte sichtbar werden kann.

Die erfindungsgemäße Brustprothese hat vorzugsweise einen oberen Ausläufer und zwei seitliche Ausläufer, nämlich einen linken Ausläufer und einen rechten Ausläufer. Betrachtet man die Rückseite der Brustprothese, so ist der links angeordnete seitliche Ausläufer der linke Ausläufer, und entsprechend der rechts angeordnete der rechte Ausläufer. Mit oberem Ausläufer ist jener Ausläufer gemeint, der im Tragezustand der Brustprothese zur Schulter der Trägerin weist. Der linke Seitenrand erstreckt sich vom oberen Ausläufer bis zum linken Ausläufer, während sich der rechte Seitenrand vom oberen Ausläufer bis zum rechten Ausläufer erstreckt. Die Brustprothese umfasst vorzugsweise einen Unterbrustrand, welcher den unteren Rand der Brustprothese bildet. Der Unterbrustrand erstreckt sich zwischen dem linken Ausläufer und dem rechten Ausläufer.

Da es sich bei der erfindungsgemäßen Brustprothese um eine symmetrische Brustprothese handelt, verläuft eine Symmetrieebene (SE) mittig und vertikal durch die Brustprothese.

Das Verhältnis Breite zu Höhe B:H liegt vorzugsweise zwischen 1,06 und 1,42, mehr bevorzugt zwischen 1,07 und 1,41, weiter mehr bevorzugt zwischen 1,10 und 1,35 und am meisten bevorzugt zwischen 1,12 und 1,31. Die Breite B liegt vorzugsweise zwischen 130 mm und 245 mm, mehr bevorzugt zwischen 140 mm und 235 mm, und die Höhe H vorzugsweise zwischen 90 mm und 230 mm, mehr bevorzugt zwischen 100 mm und 220 mm. Mit dem erfindungsgemäßen Breite B zu Höhe H Verhältnisses wird, wie oben erwähnt, eine verbesserte Passform bei zeitgleichem besserem Versorgungsausgleich erzielt.

Die Breite B ist die maximale Breite der Rückseite der Brustprothese und wird senkrecht zur vertikalen Symmetrieebene SE der Brustprothese gemessen. Sie entspricht dem Abstand zwischen den jeweils äußersten Punkten des linken Ausläufers und des rechten Ausläufers der Brustprothese.

Die Höhe H ist die maximale Höhe der Rückseite der Brustprothese und wird in bzw. entlang der symmetrischen Symmetrieebene SE der Brustprothese gemessen. Sie entspricht dem Abstand zwischen dem äußersten, oder obersten, Punktes des oberen Ausläufers und dem äußersten, oder unterstem, Punktes des Unterbrustrandes der Brustprothese.

Der linke und der rechte Seitenrand der erfindungsgemäßen Brustprothese weisen jeweils einen konkaven Verlauf auf. Mit anderen Worten weist sowohl der linke als auch der rechte Seitenrand einen nach innen gekrümmten Verlauf auf.

Der linke Seitenrand und der rechte Seitenrand der erfindungsgemäßen Brustprothese weist jeweils eine Konkavität s zwischen 0,5 mm und 6 mm, vorzugsweise zwischen 1 mm und 5 mm, mehr bevorzugt zwischen 1,5 mm und 4,5 mm, und am meisten bevorzugt zwischen 2 mm und 4 mm auf. Es ist einsichtig, dass aufgrund der symmetrischen Form der erfindungsgemäßen Brustprothese die Konkavität s des linken Seitenrandes stets der Konkavität s des rechten Seitenrandes entspricht.

Um die Konkavität s eines konkaven Seitenrandes zu messen, wird eine gedachte Referenzlinie (RL) an diesen Seitenrand gelegt. Im Falle eines konkaven Verlaufs des Seitenrandes berührt diese Referenzlinie (RL) den Seitenrand in zwei Berührpunkten. Mit Konkavität s ist der größte Abstand des Seitenrandes entlang seines Verlaufes von dieser angelegten Referenzlinie (RL) gemeint.

Es ist selbstredend, dass bei einem geradlinigen Verlauf des Seitenrandes einer Brustprothese der Verlauf des Seitenrandes genau der Referenzlinie RL entspricht, und die Konkavität s daher genau Null beträgt.

Mit der Konkavität kann auch der "Taillierungsgrad" der Brustprothese bezeichnet werden. Je größer die Konkavität s, umso konkaver verlaufen die beiden Seitenränder und desto höher ist der Taillierungsgrad der Brustprothese. Dies ist, wie oben erwähnt, vorteilhaft bei Büstenhalter mit tiefem Dekollete oder wenn die Prothese im Büstenhalter zu einem gewissen Grad gedreht werden soll.

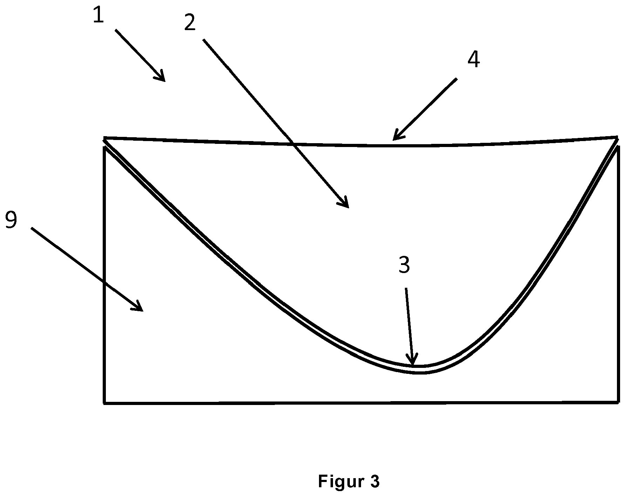

Es ist sowohl für die Messung der Breite B, der Höhe H und der Konkavität s der Brustprothese unerheblich, ob die Rückseite der Brustprothese plan oder gekrümmt ist, oder ob sie eine Vertiefung oder dergleichen aufweist. Sowohl die Breite B, die Höhe H als auch die Konkavität s werden an der Rückseite im entspannten Zustand der Brustprothese gemessen wie oben beschrieben. Mit entspanntem Zustand ist jener Zustand gemeint, den die Brustprothese einnimmt wenn sie mit Ihrer Vorderseite nach unten in ihrer Guss- oder Halteform liegt und lediglich die Schwerkraft auf sie einwirkt. Dabei ist die Guss- oder Halteform an ihrer Innenseite entsprechend der Form und Größe der Vorderseite der Brustprothese geformt, sodass die Guss- oder Halteform die Vorderseite der Brustprothese vollflächig aufnimmt, und lediglich die Schwerkraft auf die Brustprothese einwirkt, siehe Figur 3.

Vorzugsweise hat die Brustprothese eine dreiecksähnliche Form und weist einen oberen Ausläufer einen linken Ausläufer und einen rechten Ausläufer auf. Der linke und der rechte Ausläufer können auch als seitliche Ausläufer bezeichnet werden. Aufgrund der Symmetrie der Brustprothese entsprechen die beiden seitlichen Ausläufer einander spiegelbildlich in Form und Größe.

Die Ränder des oberen Ausläufers, des linken Ausläufers und des rechten Ausläufers weisen vorzugsweise jeweils einen runden Verlauf auf, insbesondere jeweils einen runden, konvexen Verlauf auf.

Der obere Ausläufer weist vorzugsweise einen Rand auf, wobei zumindest ein Teil des Verlaufs des Randes des oberen Ausläufers entspricht einem Kreisbogen. Dieser Kreisbogen entspricht vorzugsweise der Kreislinie eines Kreises mit dem Radius r_{O}, wobei r_{O} vorzugsweise zwischen 5 mm und 60 mm, mehr bevorzugt zwischen 7,5 mm und 50 mm, mehr bevorzugt zwischen 10 mm und 40 mm, und am meisten bevorzugt zwischen 14 und 35 mm beträgt. Ein solcher Radius r_{O} führt zu einem schmäleren oberen Ausläufer der Brustprothese mit den oben erwähnten überraschenden Vorteilen.

Der Kreis mit dem Radius r_{O} hat einen Mittelpunkt M_{O}. Aufgrund der symmetrischen Form der erfindungsgemäßen Brustprothese liegt der Mittelpunkt M_{O} des Kreises mit dem Radius r_{O} auf der vertikalen Symmetrieebene (SE) der Brustprothese.

Die Rückseite der Brustprothese hat vorzugsweise ein Verhältnis B:r_{O}. Dabei liegt das Verhältnis B:r_{O} vorzugsweise zwischen 5 und 15, mehr bevorzugt zwischen 5,5 und 14, mehr bevorzugt zwischen 6 und 11, und am meisten bevorzugt zwischen 6,5 und 10,5. Es wurde überraschend gefunden, dass ein in diesen Bereichen liegendes Verhältnis B:r_{O} zu einer verbesserten Passform und zu einem höheren Tragekomfort bei zeitgleich erforderlicher struktureller Stabilität der Brustprothese führt.

Wie oben erwähnt weist die Rückseite der Brustprothese vorzugsweise einen Unterbrustrand auf. Dabei entspricht vorzugsweise zumindest ein Teil des Verlaufs des Unterbrustrandes einem Kreisbogen. Dieser Kreisbogen entspricht der Kreislinie eines Kreises mit dem Radius r_{U}, wobei r_{U} vorzugsweise zwischen 100 mm und 300 mm, mehr bevorzugt zwischen 110 mm und 290 mm, mehr bevorzugt zwischen 120 mm und 280 mm, und am meisten bevorzugt zwischen 130 mm und 270 mm beträgt.

Vorzugsweise ist der Radius r_{U} größer als der Radius r_{O}, mehr bevorzugt ist der Radius r_{U} mindestens um einen Faktor 2 größer als der Radius r_{O}, mehr bevorzugt ist der Radius r_{U} mindestens um einen Faktor 3 größer als der Radius r_{O}, mehr bevorzugt ist der Radius r_{U} mindestens um einen Faktor 4 größer als der Radius r_{O}, und am meisten bevorzugt ist der Radius r_{U} mindestens um einen Faktor 5 größer als der Radius r_{O}. Üblicherweise ist der Radius r_{U} maximal um einen Faktor 10 größer als der Radius r_{O}.

Der Kreis mit dem Radius r_{U} hat einen Mittelpunkt M_{U}. Der Mittelpunkt M_{U} des Kreises mit dem Radius r_{U} liegt auf einer vertikalen Symmetrieebene (SE) der Brustprothese.

Vorzugsweise ist der Mittelpunkt M_{U} des Kreises mit dem Radius r_{U} auch der Mittelpunkt M_{O} des Kreises mit dem Radius r_{O}.

Vorzugsweise umfasst der Körper der Brustprothese eine oder mehrere Füllmassen. Die Füllmasse umfasst vorzugsweise eine weich-elastische Silikonkautschukmasse. Der Körper der Brustprothese ist vorzugsweise in einer Kunststofffolie eingeschlossen. Die Kunststofffolie ist hier vorzugsweise eine Polyurethanfolie.

Der Körper der Brustprothese kann aus einer oder mehreren Kammern bestehen, wobei die Kammern durch Trennwände, wie beispielsweise Kunststofffolien, insbesondere Polyurethanfolien, getrennt sind. Die Kammern können hier mit gleicher oder unterschiedlicher Füllmasse gefüllt sein, um die Schwingungseigenschaften der Brustprothese jener der gesunden Brust anzupassen.

Vorzugsweise ist in der Rückseite der Brustprothesen mindestens eine quer zur vertikalen Symmetrieebene verlaufende Kerbe ausgebildet. Die mindestens eine quer zur vertikalen Symmetrieebene verlaufende Kerbe ermöglicht im Tragezustand der Brustprothese bei aufrechter Haltung der Trägerin ein kontrolliertes Absacken der Brustprothese. Befindet sich die Trägerin in liegender Haltung, so ermöglicht die eine oder mehrere Kerben ein Strecken der Brustprothese. Mit der mindestens einen Kerbe wird somit ein natürliches Verhalten einer gesunden Brust nachgeahmt.

Weitere Einzelheiten, Merkmale und Vorteile des Gegenstands der Erfindung ergeben sich aus der nachfolgenden Beschreibung der zugehörigen Figuren, in welchen beispielhaft bevorzugte Ausführungsformen der Erfindung dargestellt sind.

### Darin zeigen

Figur 1 eine Seitenansicht einer Ausführungsform der erfindungsgemäßen Brustprothese,
Figur 2 die Rückansicht aus der in Figur 1 abgebildeten Ausführungsform der erfindungsgemäßen Brustprothese, und
Figur 3 eine Seitenansicht der in Figur 1 abgebildeten Ausführungsform der erfindungsgemäßen Brustprothese, welche im entspannten Zustand in Ihrer Halteform liegt.

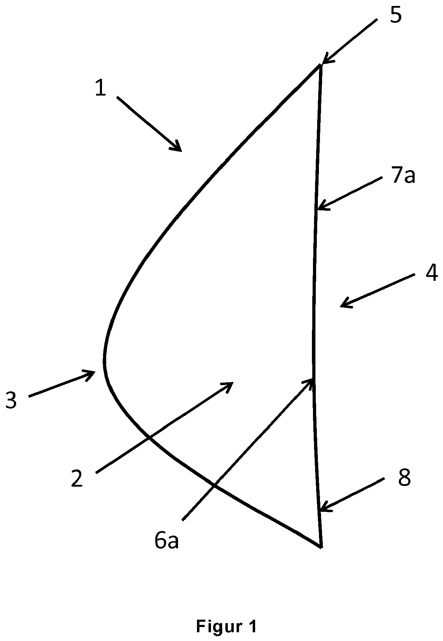

Figur 1 zeigt eine Seitenansicht einer Ausführungsform der erfindungsgemäßen Brustprothese (1), welche einen Körper (2) umfasst, der eine die Form einer weiblichen Brust aufweisende Vorderseite (3) und eine Rückseite (4) aufweist.

Der Körper (2) besteht in dieser Ausführungsform im Wesentlichen aus einer Silikon-Kautschukmasse, die in einem Beutel aus Polyurethanfolie eingeschweißt ist.

Aus Figur 1 sind weiter der linke Seitenrand (7a) ersichtlich, der sich vom oberen Ausläufer (5) bis zum linken Ausläufer (6a) erstreckt. Der Unterbrustrand (8) bildet den unteren Rand der Brustprothese (1) und erstreckt sich zwischen dem linken Ausläufer (6a) und dem rechten Ausläufer (6b).

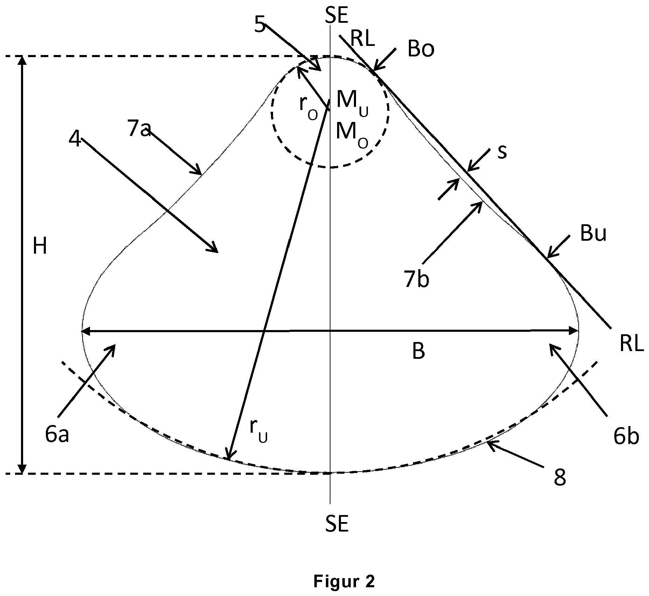

Figur 2 zeigt die Rückansicht aus der in Figur 1 abgebildeten Ausführungsform der erfindungsgemäßen Brustprothese (1). Die Brustprothese (1) hat eine Rückseite (4) mit dreiecksähnlicher Form, sowie einen oberen Ausläufer (5) und zwei seitliche Ausläufer (6a, 6b). Sowohl der obere Ausläufer (5) als auch der linke Ausläufer (6a) und der rechte Ausläufer (6b) weisen jeweils Ränder mit einem runden, konvexen Verlauf auf. Die Symmetrieebene (SE) verläuft mittig und vertikal durch die Brustprothese (1).

Die Breite B der Brustprothese (1) ist in diesem Ausführungsbeispiel 176 mm, und die Höhe H 146 mm, was einem Verhältnis B:H von 1,205 entspricht.

Wie aus Figur 2 ersichtlich wird der Verlauf des Randes des oberen Ausläufers (5) teilweise durch einen Kreisbogen eines Kreises mit dem Mittelpunkt M_{O} und dem Radius r_{O} gleich 21 mm bestimmt. Der Kreis mit dem Mittelpunkt M_{O} wird durch eine gestrichelte Linie in Figur 2 gezeigt. Da der obere Radius r_{O} 21 mm ist, beträgt das Verhältnis B:r_{O} daher 8,38.

Der Verlauf des Unterbrustrandes (8) wird ebenfalls teilweise durch einen Kreisbogen eines Kreises mit dem Mittelpunkt M_{U} und dem Radius r_{U} gleich 153 mm bestimmt. Ein Teilbereich des Kreises mit dem Mittelpunkt M_{U} wird durch eine gestrichelte Linie in Figur 2 gezeigt. Sowohl M_{O} als auch M_{U} liegen auf der vertikalen Symmetrieebene (SE) der Prothese (1), im Bereich des oberen Ausläufers (5).

Die Konkavität s wird beispielhaft am rechten Seitenrand (7b) der Brustprothese (1) gezeigt. Wie bereits oben erläutert wird eine gedachte Referenzlinie (RL) an den konkaven rechten Seitenrand (7b) der Brustprothese (1) gelegt. In diesem Ausführungsbeispiel berührt die Referenzlinie (RL) den rechten Seitenrand (7b) in zwei Berührpunkten, nämlich in einem oberen Berührpunkt Bo im Bereich des oberen Ausläufers (5) und in einem unteren Berührpunkt Bu im Bereich des rechten Ausläufers (6b). Beim oberen Berührpunkt Bo als auch beim unteren Berührpunkt Bu geht in diesem Ausführungsbeispiel der konkave Verlauf des rechten Seitenrandes (7b) in den runden, konvexen Verlauf des Randes des oberen Ausläufers (5) beziehungsweise des rechten Ausläufers (6b) über. Die Konkavität s ist der größte Abstand des Seitenrandes (7b) entlang seines Verlaufs von der angelegten Referenzlinie (RL). Wie aus Figur 2 ersichtlich, befindet sich in diesem Ausführungsbeispiel der größte Abstand zur Referenzlinie (RL) mittig auf dem Seitenrand (7b). Die Konkavität s beträgt in diesem Ausführungsbeispiel 3 mm.

Die obige Bestimmung gilt analog für den linken Seitenrand (7a). Da die erfindungsgemäße Brustprothese (1) symmetrisch ist, weist der linke Seitenrand (7a) ebenfalls eine Konkavität s von 3 mm auf.

Die hier beispielhaft beschriebene Ausführungsform ist eine im Gewicht reduzierte Brustprothese, die sowohl eine verbesserte Passform als auch die erforderliche Stabilität sowie zugleich das natürliche Schwingungsverhalten einer gesunden Brust aufweist.

Figur 3 zeigt schematisch eine Seitenansicht der in Figur 1 abgebildeten Ausführungsform der erfindungsgemäßen Brustprothese (1), welche im entspannten Zustand in Ihrer Halteform (9) liegt. In diesem entspannten Zustand wird, wie oben beschrieben, die Breite B, die Höhe H und die Konkavität s der Brustprothese (1) gemessen. Die Halteform (9) ist hier beispielsweise aus Kunststoff gefertigt. Die Prothese (1) liegt mit ihrer Vorderseite nach unten in der Halteform (9), die Rückseite (4) zeigt nach oben. Die Halteform (9) ist an ihrer Innenseite entsprechend der Form und Größe der Vorderseite (3) geformt, sodass die Halteform (9) die Vorderseite (3) der Prothese (1) vollflächig aufnimmt, und lediglich die Schwerkraft auf die Prothese (1) einwirkt.

Alternativ kann eine Gussform (9), beispielsweise aus Aluminium, genommen werden, die ebenfalls an ihrer Innenseite entsprechend der Form und Größe der Vorderseite (3) geformt ist, sodass die Gussform (9) die Vorderseite (3) der Prothese (1) vollflächig aufnimmt, und lediglich die Schwerkraft auf die Prothese (1) einwirkt, wie in Figur 3 gezeigt.

### Bezugszeichenliste

- 1: Brustprothese
- 2: Körper der Brustprothese (1)
- 3: Vorderseite der Brustprothese (1)
- 4: Rückseite der Brustprothese (1)
- 5: Oberer Ausläufer
- 6a: Linker Ausläufer
- 6b: Rechter Ausläufer
- 7a: Linker Seitenrand
- 7b: Rechter Seitenrand
- 8: Unterbrustrand
- 9: Guss- oder Halteform
- B: Breite der Rückseite (4)
- Bo: oberer Berührpunkt der Referenzlinie (RL)
- Bu: unterer Berührpunkt der Referenzlinie (RL)
- H: Höhe der Rückseite (4)
- RL: Referenzlinie
- r_{O}: oberer Radius
- r_{U}: Unterbrustradius
- s: Konkavität des linken Seitenrandes (7a) und rechten Seitenrandes (7b)
- SE: Symmetrieebene

## Patentansprüche

1. Symmetrische Brustprothese (1) umfassend einen Körper (2), der eine die Form einer weiblichen Brust aufweisende Vorderseite (3) und eine Rückseite (4) aufweist, wobei die Rückseite (4) einen linken Seitenrand (7a), einen rechten Seitenrand (7b), eine Breite B und eine Höhe H aufweist, **dadurch gekennzeichnet, dass**
das Verhältnis B:H zwischen 1,05 und 1,45 liegt, und dass
der linke Seitenrand (7a) und der rechte Seitenrand (7b) jeweils einen konkaven Verlauf aufweisen und jeweils eine Konkavität s zwischen 0,5 mm und 6 mm aufweisen.

2. Brustprothese nach Anspruch 1, **dadurch gekennzeichnet, dass** die Brustprothese (1) eine dreiecksähnliche Form hat und einen oberen Ausläufer (5), einen linken Ausläufer (6a) und einen rechten Ausläufer (6b) aufweist.

3. Brustprothese nach Anspruch 2, **dadurch gekennzeichnet, dass** der obere Ausläufer (5) einen Rand aufweist und zumindest ein Teil des Verlaufs des Randes des oberen Ausläufers (5) einem Kreisbogen entspricht.

4. Brustprothese nach Anspruch 3, **dadurch gekennzeichnet, dass** der Kreisbogen der Kreislinie eines Kreises mit dem Radius r_{O} entspricht.

5. Brustprothese nach Anspruch 4, **dadurch gekennzeichnet, dass** der Radius r_{O} zwischen 5 mm und 60 mm beträgt.

6. Brustprothese nach Anspruch 4 oder 5, **dadurch gekennzeichnet, dass** die Rückseite (4) ein Verhältnis B:r_{O} hat, wobei das Verhältnis B:r_{O} zwischen 5 und 15 liegt.

7. Brustprothese nach einem der vorhergehenden Ansprüche, **dadurch gekennzeichnet, dass** die Rückseite (4) einen Unterbrustrand (8) aufweist.

8. Brustprothese nach Anspruch 7, **dadurch gekennzeichnet, dass** zumindest ein Teil des Verlaufs des Unterbrustrandes (8) einem Kreisbogen entspricht.

9. Brustprothese nach Anspruch 8, **dadurch gekennzeichnet, dass** der Kreisbogen der Kreislinie eines Kreises mit dem Radius r_{U} entspricht.

10. Brustprothese nach Anspruch 9, **dadurch gekennzeichnet, dass** der Radius r_{U} zwischen 100 mm und 300 mm beträgt.

11. Brustprothese nach Anspruch 10, **dadurch gekennzeichnet, dass** der Mittelpunkt M_{U} des Kreises mit dem Radius r_{U} gleichzeitig der Mittelpunkt M_{O} des Kreises mit dem Radius r_{O} ist.

12. Brustprothese nach Anspruch 10, **dadurch gekennzeichnet, dass** der Mittelpunkt M_{O} des Kreises mit dem Radius r_{O} im Bereich des oberen Ausläufers (5) auf einer vertikalen Symmetrieebene (SE) der Brustprothese (1) liegt.

13. Brustprothese nach einem der vorhergehenden Ansprüche, **dadurch gekennzeichnet, dass** der Körper (2) eine Füllmasse umfasst.

14. Brustprothese nach Anspruch 13, **dadurch gekennzeichnet dass** die Füllmasse eine weich-elastische Silikonkautschukmasse umfasst.

15. Brustprothese nach einem der vorhergehenden Ansprüche, **dadurch gekennzeichnet, dass** in der Rückseite (4) der Brustprothese (1) mindestens eine quer zur vertikalen Symmetrieebene (SE) verlaufende Kerbe ausgebildet ist.
